(19) Europäisches Patentamt / European Patent Office / Office européen des brevets

(11) **EP 4 306 092 A1**

(12) **EUROPEAN PATENT APPLICATION**
published in accordance with Art. 153(4) EPC

(43) Date of publication:
**17.01.2024  Bulletin 2024/03**

(21) Application number: **22780191.7**

(22) Date of filing: **17.03.2022**

(51) International Patent Classification (IPC):
**A61F 13/53** (2006.01)

(52) Cooperative Patent Classification (CPC):
**A61F 13/53**

(86) International application number:
**PCT/JP2022/012368**

(87) International publication number:
**WO 2022/209972 (06.10.2022 Gazette 2022/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.03.2021   JP 2021056566**

(71) Applicant: **Sumitomo Seika Chemicals Co., Ltd.
Kako-gun, Hyogo 675-0145 (JP)**

(72) Inventors:
• **YAMAMOTO, Tomoka
  Himeji-shi, Hyogo 672-8076 (JP)**
• **SAWAKI, Hiroki
  Himeji-shi, Hyogo 672-8076 (JP)**

(74) Representative: **Vossius & Partner
Patentanwälte Rechtsanwälte mbB
Siebertstrasse 3
81675 München (DE)**

(54)   **ABSORBER**

(57)   The present invention relates to an absorber containing an absorber core having water-absorbent resin particles, in which a diffusion area change ratio measured in the order of (1) to (5) is 0.80 to 1.0.

## Fig.1

**Description**

**Technical Field**

The present invention relates to an absorber.

**Background Art**

In the related art, an absorber containing water-absorbent resin particles has been used in an absorbent article for absorbing a liquid having water as a main component such as urine. For example, Patent Literature 1 discloses a method for producing water-absorbent resin particles having a particle diameter that enables them to be suitably used for absorbent articles such as diapers.

**Citation List**

**Patent Literature**

[Patent Literature 1] Japanese Unexamined Patent Publication No. H06-345819

**Summary of Invention**

**Technical Problem**

By improving the utilization of an absorber in an absorbent article to use the absorbent article comfortably for a longer period of time, the frequency of replacement of absorbent articles such as diapers in childcare or nursing care is reduced, and it is expected that the burden on consumers will be reduced and environmental loads will be reduced.
Furthermore, absorbers are generally formed by mixing water-absorbent resin particles with a fibrous material such as pulp, but as absorbers become thinner, absorbers with an increased use proportion of water-absorbent resin particles are also used. However, the absorption rate of an absorber tends to be high when the use proportion of a fibrous material in the absorber is high to some extent.
An object of the present invention is to provide an absorber having a better absorption rate even when the content of water-absorbent resin particles is the same in the total amount of the water-absorbent resin particles and a fibrous material in the absorber.

**Solution to Problem**

An absorber of the present invention contains an absorber core containing water-absorbent resin particles, in which a diffusion area change ratio measured in the order of the following (1) to (5) is 0.80 to 1.0.

(1) An absorbent article is produced by disposing, on an upper surface of the absorber, an air-through type polyethylene porous liquid permeable sheet having a basis weight of 22 $g/m^2$ and having the same area as that of the absorber.
(2) The absorbent article is placed horizontally, a cylinder having an opening with an inner diameter of 3 cm is placed on a center of the absorbent article, 80 ml of artificial urine is injected into the cylinder at one time, and the cylinder is removed.
(3) An area $S_1$ of a surface of the absorber on which the artificial urine is diffused is measured 20 minutes after the artificial urine injection.
(4) A second artificial urine injection is performed in the same manner as in (2) 30 minutes after the first artificial urine injection, and a total area $S_2$ of the surface of the absorber on which the artificial urine is diffused is measured 20 minutes after the second artificial urine injection.
(5) The operation of (4) is repeated twice more to measure a diffusion area $S_3$ at the time of a third injection and a diffusion area $S_4$ at the time of a fourth injection.

$$\text{Diffusion area change ratio} = (S_1 + S_2)/(S_3 + S_4)$$

An effective diffusion area fraction of the absorber is preferably 95% or less.

$$\text{Effective diffusion area fraction (\%)} = (S_4/\text{area of absorber core})$$

$$\times 100$$

A content of the water-absorbent resin particles may be 5% to 100% by mass with respect to a total amount of the water-absorbent resin particles and a fibrous material in the absorber.

In the above-mentioned absorber, the water-absorbent resin particles preferably include water-absorbent resin particles A in which a 2-minute value of a water absorption rate of physiological saline is 2.5 cm or less, and a content of the water-absorbent resin particles A is preferably 9% to 65% by mass with respect to a total amount of the water-absorbent resin particles in the absorber.

The water-absorbent resin particles preferably include water-absorbent resin particles B in which a 2-minute value of a water absorption rate of physiological saline is 4.0 cm or more.

A water retention capacity of physiological saline of the water-absorbent resin particles A is preferably 16 to 55 g/g.

The water-absorbent resin particles A are preferably coated resin particles having a water-insoluble coating layer that coats at least a part of a surface of crosslinked polymer particles.

**Advantageous Effects of Invention**

According to the present invention, an absorber having a better absorption rate even when the content of water-absorbent resin particles is the same in the total amount of the water-absorbent resin particles and a fibrous material in the absorber is provided.

**Brief Description of Drawings**

Fig. 1 is a schematic cross-sectional view showing one embodiment of an absorbent article.
Fig. 2 is a schematic view showing a method for evaluating leakage of the absorbent article.

**Description of Embodiments**

Hereinafter, some embodiments of the present invention will be described in detail. However, the present invention is not limited to the following embodiments.

In the present specification, "acrylic" and "methacrylic" are collectively referred to as "(meth)acrylic". Similarly, "acrylate" and "methacrylate" are also referred to as "(meth)acrylate". "(Poly)" means both of a case where there is a prefix of "poly" and a case where there is no prefix thereof. In a numerical value range described in stages in the present specification, the upper limit value or the lower limit value of the numerical value range of a stage can be optionally combined with the upper limit value or the lower limit value of the numerical value range of another stage. In a numerical value range described in the present specification, the upper limit value or the lower limit value of the numerical value range may be replaced with the value shown in Examples. For materials exemplified in the present specification, one type may be used alone, or two or more types may be used in combination. The content of each component in the composition means the total amount of a plurality of substances present in the composition in a case where the plurality of substances corresponding to each component are present in the composition, unless otherwise specified. "Physiological saline" refers to an aqueous solution of 0.9% by mass sodium chloride. The term "layer" includes not only a structure having a shape formed on the entire surface when observed as a planar view, but also a structure having a shape partially formed.

An absorber of the present embodiment contains an absorber core containing water-absorbent resin particles, in which a diffusion area change ratio is 0.80 to 1.0. The diffusion area change ratio is measured in the order of the following (1) to (5).

(1) An absorbent article is produced by disposing, on an upper surface of the absorber, an air-through type polyethylene porous liquid permeable sheet having a basis weight of 22 g/m$^2$ and having the same area as that of the absorber.

(2) The absorbent article is placed horizontally, a cylinder having an opening with an inner diameter of 3 cm is placed on a center of the absorbent article, 80 ml of artificial urine is injected into the cylinder at one time, and the cylinder is removed.

(3) An area $S_1$ of a surface of the absorber on which the artificial urine is diffused is measured 20 minutes after the artificial urine injection.

(4) A second artificial urine injection is performed in the same manner as in (2) 30 minutes after the first artificial urine injection, and a total area $S_2$ of the surface of the absorber on which the artificial urine is diffused is measured 20 minutes after the second artificial urine injection.

(5) The operation of (4) is repeated twice more to measure a diffusion area $S_3$ at the time of a third injection and a diffusion area $S_4$ at the time of a fourth injection.

$$\text{Diffusion area change ratio} = (S_1 + S_2)/(S_3 + S_4)$$

In the absorber of the present embodiment, since the diffusion area change ratio is set to 0.80 to 1.0, an absorption rate can be further increased without changing the content of the water-absorbent resin particles in the total amount of the water-absorbent resin particles and a fibrous material in the absorber.

A theoretical maximum value of the diffusion area change ratio is 1. As the diffusion area change ratio becomes higher, this means that the diffuseness in the first two times of water absorption among repeated water absorption becomes better, and further diffusion in the subsequent two times of water absorption becomes lesser. Therefore, it can be said that, as the diffusion area change ratio becomes higher, a liquid to be absorbed is more likely to diffuse into a wider area of the absorber at the initial stage of use of the absorber, thereby making it possible to more effectively utilize the absorber. In addition, the inventors of the present invention have found for the first time that a high diffusion area change ratio contributes to a better absorption rate. The absorber of the present embodiment has a better absorption rate because the diffuseness after absorbing a liquid to be absorbed a plurality of times is minimized.

The diffusion area change ratio of the absorber may be 0.81 or more, 0.82 or more, 0.83 or more, 0.84 or more, 0.85 or more, 0.86 or more, 0.87 or more, or 0.88 or more, and may be 1.0 or less, 0.95 or less, 0.92 or less, 0.90 or less, 0.88 or less, 0.86 or less, 0.84 or less, or 0.82 or less.

In the absorber, an effective diffusion area fraction represented by the following formula is preferably 95% or less.

$$\text{Effective diffusion area fraction (\%)} = (S_4/\text{area of absorber core}) \times 100$$

In the absorber of the present embodiment, leakage can be prevented by setting the effective diffusion area fraction to 95% or less, in addition to setting of the diffusion area change ratio to 0.80 to 1.0.

The effective diffusion area fraction (ratio of $S_4$ to the area of the absorber core) may be 93% or less, 90% or less, 88% or less, 85% or less, 83% or less, 80% or less, 78% or less, 77% or less, 75% or less, 73% or less, or 70% or less. Furthermore, the effective diffusion area fraction is preferably 60% or more, and may be 62% or more, 63% or more, 65% or more, 68% or more, 70% or more, 73% or more, 75% or more, 78% or more, 80% or more, 83% or more, 85% or more, 88% or more, or 90% or more. It is shown that the higher the effective diffusion area fraction is, the more widely the absorber core can be utilized. When the effective diffusion area fraction is within the above-mentioned range, the utilization of the absorber core is increased, making it possible to use the absorber for a longer period of time, which reduces the frequency of replacement of diapers in childcare or nursing care, and thereby it is expected that the burden on users will be reduced and leakage will be prevented. In addition, a reduction in the consumed amount of absorbent articles such as diapers can contribute to a reduction in environmental loads.

$S_1$ may be 200 cm$^2$ or more, 210 cm$^2$ or more, 220 cm$^2$ or more, 240 cm$^2$ or more, 260 cm$^2$ or more, or 270 cm$^2$ or more, and may be 350 cm$^2$ or less, 340 cm$^2$ or less, 330 cm$^2$ or less, 320 cm$^2$ or less, 310 cm$^2$ or less, 300 cm$^2$ or less, 290 cm$^2$ or less, 280 cm$^2$ or less, 270 cm$^2$ or less, 260 cm$^2$ or less, 250 cm$^2$ or less, 240 cm$^2$ or less, or 220 cm$^2$ or less.

$S_2$ may be 240 cm$^2$ or more, 250 cm$^2$ or more, 260 cm$^2$ or more, 270 cm$^2$ or more, 280 cm$^2$ or more, or 300 cm$^2$ or more, and may be 370 cm$^2$ or less, 360 cm$^2$ or less, 350 cm$^2$ or less, 330 cm$^2$ or less, 310 cm$^2$ or less, 290 cm$^2$ or less, 280 cm$^2$ or less, 270 cm$^2$ or less, 260 cm$^2$ or less, or 250 cm$^2$ or less.

$S_3$ may be 250 cm$^2$ or more, 260 cm$^2$ or more, 270 cm$^2$ or more, 280 cm$^2$ or more, 290 cm$^2$ or more, or 300 cm$^2$ or more, and may be 400 cm$^2$ or less, 390 cm$^2$ or less, 380 cm$^2$ or less, 370 cm$^2$ or less, 360 cm$^2$ or less, 350 cm$^2$ or less, 340 cm$^2$ or less, 320 cm$^2$ or less, 310 cm$^2$ or less, 300 cm$^2$ or less, 290 cm$^2$ or less, 280 cm$^2$ or less, or 270 cm$^2$ or less.

$S_4$ may be 280 cm$^2$ or more, 290 cm$^2$ or more, 300 cm$^2$ or more, 310 cm$^2$ or more, 320 cm$^2$ or more, 340 cm$^2$ or more, 360 cm$^2$ or more, or 380 cm$^2$ or more, and may be 480 cm$^2$ or less, 470 cm$^2$ or less, 460 cm$^2$ or less, 440 cm$^2$ or less, 420 cm$^2$ or less, 400 cm$^2$ or less, 380 cm$^2$ or less, 360 cm$^2$ or less, 340 cm$^2$ or less, 330 cm$^2$ or less, 320 cm$^2$

or less, 310 cm$^2$ or less, or 300 cm$^2$ or less.

The total of S$_1$ and S$_2$ may be 420 cm$^2$ or more, 430 cm$^2$ or more, 450 cm$^2$ or more, 470 cm$^2$ or more, 490 cm$^2$ or more, 510 cm$^2$ or more, 530 cm$^2$ or more, or 550 cm$^2$ or more, and may be 680 cm$^2$ or less, 660 cm$^2$ or less, 640 cm$^2$ or less, 620 cm$^2$ or less, 600 cm$^2$ or less, 580 cm$^2$ or less, 560 cm$^2$ or less, 540 cm$^2$ or less, 520 cm$^2$ or less, 500 cm$^2$ or less, or 490 cm$^2$ or less.

The total of S$_3$ and S$_4$ may be 550 cm$^2$ or more, 560 cm$^2$ or more, 580 cm$^2$ or more, 600 cm$^2$ or more, 620 cm$^2$ or more, 630 cm$^2$ or more, 640 cm$^2$ or more, 660 cm$^2$ or more, 680 cm$^2$ or more, or 700 cm$^2$ or more, and may be 900 cm$^2$ or less, 880 cm$^2$ or less, 860 cm$^2$ or less, 840 cm$^2$ or less, 800 cm$^2$ or less, 760 cm$^2$ or less, 720 cm$^2$ or less, 680 cm$^2$ or less, 640 cm$^2$ or less, 630 cm$^2$ or less, 600 cm$^2$ or less, or 580 cm$^2$ or less. From the viewpoint of being able to prevent leakage while having a better absorption rate, each of S$_1$, S$_2$, S$_3$, S$_4$, the total of S$_1$ and S$_2$, and the total of S$_3$ and S$_4$ may be in the above-mentioned range.

[Water-absorbent resin particles A]

The water-absorbent resin particles used for the absorber preferably include water-absorbent resin particles A in which a 2-minute value of a water absorption rate of physiological saline is 2.5 cm or less. When the water-absorbent resin particles include the water-absorbent resin particles A in which the water absorption rate is reduced to a certain level or less, the diffuseness of the absorber at the initial stage of water absorption becomes high, and the diffusion area change ratio of the absorber is easily adjusted to a suitable range, which is advantageous for preventing leakage and improving the absorption rate of the absorber.

The 2-minute value of the water absorption rate of physiological saline of the water-absorbent resin particles A may be 2.2 cm or less, 2.0 cm or less, 1.8 cm or less, 1.5 cm or less, 1.3 cm or less, 1.0 cm or less, 0.8 cm or less, 0.5 cm or less, or 0.3 cm or less, and may be 0.1 cm or more, 0.2 cm or more, 0.3 cm or more, or 0.4 cm or more.

The 2-minute value of the water absorption rate of physiological saline is measured by the following procedure. 0.200 g of the particles is weighed exactly and spread on the bottom of an acrylic cylinder with an inner diameter of 2.0 cm and a depth of 8.0 cm to measure a height H$_0$ of a particle layer. Thereafter, 20 g of physiological saline is weighed and poured into the acrylic cylinder from its upper part. A height H$_2$ of the particle layer is read after 2 minutes from the time when the total amount of the physiological saline has been put. The 2-minute value of the water absorption rate is calculated from the following formula.

$$\text{2-Minute value of water absorption rate (cm)} = (H_2 - H_0)$$

From the viewpoint of further improving the absorption rate of the absorber and facilitating the prevention of leakage, the water retention capacity of physiological saline of the water-absorbent resin particles A may be 16 g/g or more, 20 g/g or more, 25 g/g or more, 30 g/g or more, 32 g/g or more, 34 g/g or more, 36 g/g or more, or 38 g/g or more, and may be 55 g/g or less, 53 g/g or less, 51 g/g or less, 49 g/g or less, 47 g/g or less, 45 g/g or less, 43 g/g or less, 41 g/g or less, or 39 g/g or less. The water retention capacity of physiological saline is measured by a method described in Examples to be described later.

The content of the water-absorbent resin particles A is preferably 9% to 65% by mass with respect to the total amount of the water-absorbent resin particles in the absorber from the viewpoint of further improving the absorption rate of the absorber and facilitating the prevention of leakage. The content of the water-absorbent resin particles A with respect to the total amount of the water-absorbent resin particles in the absorber may be 10% by mass or more, 12% by mass or more, 15% by mass or more, 18% by mass or more, 20% by mass or more, 25% by mass or more, 30% by mass or more, 35% by mass or more, 40% by mass or more, 45% by mass or more, 50% by mass or more, 55% by mass or more, or 60% by mass or more, and may be 65% by mass or less, 63% by mass or less, 60% by mass or less, 55% by mass or less, 50% by mass or less, 45% by mass or less, 40% by mass or less, 35% by mass or less, 30% by mass or less, 25% by mass or less, 20% by mass or less, or 15% by mass or less.

For example, the water-absorbent resin particles A may be coated resin particles having crosslinked polymer particles, and a water-insoluble coating layer that coats at least a part of the surface of the crosslinked polymer particles.

The crosslinked polymer particles constituting the coated resin particles contain a resin having water absorption properties, and may contain a crosslinked polymer formed by polymerization of a monomer including an ethylenically unsaturated monomer, for example. The crosslinked polymer can have a monomer unit derived from the ethylenically unsaturated monomer. The crosslinked polymer particles can be produced by a method including polymerizing monomers including ethylenically unsaturated monomers, for example. Examples of methods of polymerization include a reverse phase suspension polymerization method, an aqueous solution polymerization method, a bulk polymerization method, and a precipitation polymerization method.

The ethylenically unsaturated monomer may be a water-soluble ethylenically unsaturated monomer (an ethylenically unsaturated monomer having the solubility of 1 g or more at 98°C in 100 g of water). Examples of the water-soluble ethylenically unsaturated monomer include (meth)acrylic acid and a salt thereof, 2-(meth)acrylamide-2-methylpropanesulfonic acid and a salt thereof, (meth)acrylamide, N,N-dimethyl (meth)acrylamide, 2-hydroxyethyl (meth)acrylate, N-methylol (meth)acrylamide, polyethylene glycol mono(meth)acrylate, N,N-diethylaminoethyl (meth)acrylate, N,N-diethylaminopropyl (meth)acrylate, and diethylaminopropyl (meth)acrylamide. When the ethylenically unsaturated monomer has an amino group, the amino group may be quaternized. The ethylenically unsaturated monomer may be used alone, or may be used in combination of two or more types thereof.

When the ethylenically unsaturated monomer has an acid group, it may be used in the polymerization reaction after neutralizing the acid group with an alkaline neutralizing agent. The degree of neutralization of the ethylenically unsaturated monomer by the alkaline neutralizing agent may be 10 to 100 mol%, 50 to 90 mol%, or 60 to 80 mol% of the acid group in the ethylenically unsaturated monomer, for example.

From the viewpoint of industrial availability, the ethylenically unsaturated monomer may contain at least one compound selected from the group consisting of (meth)acrylic acid and a salt thereof, acrylamide, methacrylamide, and N,N-dimethyl acrylamide. The ethylenically unsaturated monomer may contain at least one compound selected from the group consisting of (meth)acrylic acid and a salt thereof, and acrylamide.

For the monomer for obtaining the crosslinked polymer particles, a monomer other than the above-mentioned ethylenically unsaturated monomers may be used. Such a monomer can be used by being mixed with an aqueous solution containing the above-mentioned ethylenically unsaturated monomers, for example. The use amount of the ethylenically unsaturated monomer may be 70 to 100 mol% with respect to the total amount of monomers. The ratio of (meth)acrylic acid and a salt thereof may be 70 to 100 mol% with respect to the total amount of the monomers.

Crosslinking by self-crosslinking may occur during polymerization, but crosslinking may be promoted by using an internal crosslinking agent. When the internal crosslinking agent is used, water-absorbent characteristics (water retention capacity and the like) of the crosslinked polymer particles are easily controlled. The internal crosslinking agent is usually added to a reaction solution during the polymerization reaction.

The crosslinked polymer particles may be those in which crosslinking (surface crosslinking) is performed in the vicinity of the surface. In addition, the crosslinked polymer particles may be constituted of only the polymer particles (crosslinked polymer), but may further contain various additional components selected from a gel stabilizer, a metal chelating agent, a flowability improver (lubricant), and the like, for example. The additional component can be disposed inside the polymer particles, on the surface of the polymer particles, or both thereof. The additional component may be a flowability improver (lubricant). The flowability improver may contain inorganic particles. Examples of the inorganic particles include silica particles such as amorphous silica.

The shape of the coated resin particles is not particularly limited, and may be substantially spherical, crushed, or granular, or may be a shape in which primary particles having these shapes are aggregated, for example.

The median particle diameter of the coated resin particles may be 100 to 800 μm, 150 to 700 μm, 200 to 600 μm, or 250 to 500 μm. The median particle diameter can be measured by the following method.

[Method for measuring median particle diameter]

JIS standard sieves are combined in the order of a sieve having an opening of 850 μm, a sieve having an opening of 600 μm, a sieve having an opening of 500 μm, a sieve having an opening of 425 μm, a sieve having an opening of 300 μm, a sieve having an opening of 250 μm, a sieve having an opening of 180 μm, a sieve having an opening of 150 μm, and a tray from the top. 50 g of the water-absorbent resin particles is put in the topmost sieve among the combined sieves, and classification is performed using a Ro-tap type shaker (manufactured by Iida-seisakusho Japan Corporation) according to JIS Z 8815 (1994). After classification, the mass of the particles remaining on each sieve is calculated as a mass percentage with respect to the total amount, and the particle size distribution is obtained. The relationship between the opening of the sieve and the integrated value of the mass percentage of the particles remaining on the sieve is plotted on logarithmic probability paper by integrating in the order from the one having the largest particle diameter on the sieve with respect to this particle size distribution. By connecting the plots on the probability paper with a straight line, the particle diameter corresponding to the cumulative mass percentage of 50% by mass is obtained as the median particle diameter.

The water-insoluble coating layer constituting the coated resin particles is a layer containing a water-insoluble component. In the present specification, the water-insoluble component may include not only a substance that is completely insoluble in water but also a substance that is slightly soluble in water (a substance that is sparingly soluble in water). The solubility of the water-insoluble component in 100 g of water is less than 10 g at 98°C, preferably less than 5 g, more preferably less than 3 g, and further preferably less than 1 g, for example.

The coating layer may be a layer containing at least one water-insoluble component selected from a water-insoluble organic compound and a water-insoluble inorganic compound.

Examples of the water-insoluble organic compound include a mixed composition of a polymer containing a substituted or unsubstituted alkene as a structural unit and a polymer containing an alkylene oxide as a structural unit, a polymer containing a vinyl halide as a structural unit, polyurethane, polyester, polyamide, polystyrene, polycarbonate, polyacrylate, polyacetal, and acid-modified products thereof.

Among these, it is preferable to incorporate at least one selected from the group consisting of a mixed composition of a polymer containing a substituted or unsubstituted alkene as a structural unit and a polymer containing an alkylene oxide as a structural unit, and a polymer containing a vinyl halide as a structural unit, and it is more preferable to incorporate at least one selected from the group consisting of a mixed composition of a copolymer containing an unsubstituted alkene as a structural unit and a polymer containing an alkylene oxide as a structural unit, and a homopolymer containing a vinyl halide as a structural unit.

The copolymer, which contains an unsubstituted alkene as a structural unit and is contained in the mixed composition, may be a copolymer containing only two or more types of unsubstituted alkene as a structural unit, or may be a copolymer containing one type or two or more types of unsubstituted alkene and a monomer component other than the unsubstituted alkene as structural units. The copolymer containing an unsubstituted alkene as a structural unit is preferably a copolymer containing one type of unsubstituted alkene and a monomer component other than the unsubstituted alkene as structural units.

Examples of the unsubstituted alkenes used in the copolymer include ethylene, propylene, and butene, but ethylene and/or propylene is preferably used, and ethylene is more preferably used. In addition, as the monomer component other than the unsubstituted alkene used in the copolymer, a water-soluble ethylenically unsaturated monomer is preferably used. The above-mentioned compound can be used as the water-soluble ethylenically unsaturated monomer, but (meth)acrylic acid and/or a salt thereof is preferably used.

Overall, as the copolymer containing an alkene as a structural unit, a copolymer containing ethylene and a water-soluble ethylenically unsaturated monomer as structural units is preferable, a copolymer containing ethylene and (meth)acrylic acid and/or a salt thereof structural units is more preferable, a copolymer containing ethylene and an acrylic acid salt as structural units is further preferable, and a copolymer (ethylene-sodium acrylate copolymer) containing ethylene and sodium acrylate as structural units is particularly preferable.

The polymer, which contains an alkylene oxide as a structural unit and is contained in the mixed composition, may be a polyalkylene oxide (homopolymer) containing only one type of alkylene oxide as a structural unit, may be a polyalkylene oxide (copolymer) containing two or more types of alkylene oxide as a structural unit, or may be a copolymer containing one type or two or more types of alkylene oxide and a monomer component other than the alkylene oxide as structural units. A polyalkylene oxide containing only one type of alkylene oxide as a structural unit is preferably used.

Examples of the alkylene oxide include ethylene oxide and propylene oxide, and preferable examples thereof include ethylene oxide.

Overall, the polymer containing an alkylene oxide as a structural unit is preferably at least one selected from the group consisting of a homopolymer (polyethylene glycol) containing ethylene oxide as a structural unit, and an ethylene-propylene copolymer containing ethylene oxide and propylene oxide as structural units, among which polyethylene glycol is more preferable.

Examples of the homopolymer containing a vinyl halide as a structural unit include homopolymers containing chloroethylene, bromoethylene, or fluoroethylene as a structural unit, among which a homopolymer (polyvinyl chloride) containing chloroethylene as a structural unit is preferably used.

Examples of the water-insoluble inorganic compound include light anhydrous silicic acid, calcium silicate, silicon dioxide (silica), talc, silicon oxide, and synthetic hydrotalcite. For these inorganic compounds, one type may be used alone, or a plurality of types may be used in combination. From the viewpoint of obtaining a relatively high water permeability when the coating layer is formed, at least one of silicon dioxide and talc is preferably used, and silicon dioxide is more preferably used. The silicon dioxide may be hydrophilic or hydrophobic. The silicon dioxide is preferably hydrophobic from the viewpoint of easily adjusting the 2-minute value of the water absorption rate of the water-absorbent resin particles to an appropriate range.

When forming the coating layer on the crosslinked polymer particles, the coating layer may be formed on at least a part of the surface of the crosslinked polymer particles by mixing the crosslinked polymer particles and a coating material. The coating material is a component capable of forming the above-mentioned coating layer or a formation material of the component, for example. For example, when the coating layer contains polyurethane, the coating material may contain polyurethane itself, or may contain polyol and polyisocyanate which are formation materials of the polyurethane.

A method of forming the coating layer is not particularly limited. For example, the coating layer can be formed by bringing the crosslinked polymer particles into a dispersed state, and thereafter bringing the coating material into contact with the crosslinked polymer particles in the dispersed state. Specifically, when the coating material is dissolved in a dispersion medium by which the polymer particles are dispersed, the coating layer may be formed on the surface of the crosslinked polymer particles by adding the crosslinked polymer particles and the coating material to the dispersion medium. In addition, when polyol and polyisocyanate are used as the coating material, the coating layer containing polyurethane may be formed on the surface of the crosslinked polymer particles by mixing an aqueous solution of polyol with a dispersion liquid of the crosslinked polymer particles to bring the crosslinked polymer particles and polyol into contact with each other, and thereafter mixing a liquid containing polyisocyanate to polymerize polyol and polyisocyanate.

The dispersion medium may contain a hydrocarbon solvent. Examples of the hydrocarbon solvent include chained aliphatic hydrocarbons such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene.

When a solid-like inorganic compound is used as the coating material, the coating layer can be formed by pressure-bonding the coating material to the surface of the crosslinked polymer particles using a particle composing machine. Specifically, a predetermined amount of the crosslinked polymer particles and the solid-like inorganic compound are injected into the particle composing machine. Subsequently, stresses (compressive stress and shear stress) are applied to the crosslinked polymer particles and the inorganic compound by rotation of a stirrer blade of the machine to pressure-bond the inorganic compound to the surface of the crosslinked polymer particles by the stresses, thereby producing the coated resin particles.

As the water-absorbent resin particles A, one type may be used alone, or a plurality of types may be used in combination.

[Water-absorbent resin particles B]

The water-absorbent resin particles used for the absorber preferably include water-absorbent resin particles B in which a 2-minute value of a water absorption rate is 4.0 cm or more. When the water-absorbent resin particles include the water-absorbent resin particles B in combination with the water-absorbent resin particles A, while maintaining a high diffuseness in the absorber at the initial stage of water absorption, a better water absorption rate is exhibited after a plurality of times of water absorption, making it easy to adjust the diffusion area change ratio within a suitable range, which is advantageous for preventing the leakage and improving the absorption rate of the absorber. When the water-absorbent resin particles include the water-absorbent resin particles A and the water-absorbent resin particles B, it is preferable that both be uniformly mixed and uniformly distributed in the absorber.

The 2-minute value of the water absorption rate of the water-absorbent resin particles B may be 4.2 cm or more, or 4.5 cm or more. The 2-minute value of the water absorption rate of the water-absorbent resin particles B may be 6.5 cm or less, 5.5 cm or less, 5.2 cm or less, 5.0 cm or less, or 4.8 cm or less.

From the viewpoint of further improving the absorption rate of the absorber and facilitating the prevention of leakage, the water retention capacity of physiological saline of the water-absorbent resin particles B may be 30 g/g or more, 35 g/g or more, 38 g/g or more, or 40 g/g or more, and may be 60 g/g or less, 50 g/g or le ss, 48 g/g or less, 45 g/g or less, or 43 g/g or less.

The content of the water-absorbent resin particles B is preferably 35% to 91% by mass with respect to the total amount of the water-absorbent resin particles in the absorber from the viewpoint of further improving the absorption rate of the absorber and facilitating the prevention of leakage. The content of the water-absorbent resin particles B with respect to the total amount of the water-absorbent resin particles in the absorber may be 38% by mass or more, 40% by mass or more, 45% by mass or more, 50% by mass or more, 55% by mass or more, 60% by mass or more, 65% by mass or more, 70% by mass or more, 75% by mass or more, 80% by mass or more, 85% by mass or more, or 90% by mass or more, and may be 90% by mass or less, 85% by mass or less, 80% by mass or less, 75% by mass or less, 70% by mass or less, 65% by mass or less, 60% by mass or less, 55% by mass or less, 50% by mass or less, 45% by mass or less, or 40% by mass or less.

The water-absorbent resin particles B may include the crosslinked polymer particles formed by polymerizing a monomer including an ethylenically unsaturated monomer, for example. The crosslinked polymer particles can have a monomer unit derived from the ethylenically unsaturated monomer. The water-absorbent resin particles can be produced by a method including polymerizing a monomer including an ethylenically unsaturated monomer, for example. Examples of methods of polymerization include a reverse phase suspension polymerization method, an aqueous solution polymerization method, a bulk polymerization method, and a precipitation polymerization method.

For the crosslinked polymer particles constituting the water-absorbent resin particles B, the same aspect as that of the crosslinked polymer particles constituting the above-mentioned coated resin particles can be applied, for example. The water-absorbent resin particles B may be those that are not coated with a resin.

The water-absorbent resin particles B may be those in which crosslinking (surface crosslinking) is performed in the vicinity of the surface. In addition, the water-absorbent resin particles B may be constituted of only the polymer particles (crosslinked polymer), but may further contain various additional components selected from a gel stabilizer, a metal chelating agent, a flowability improver (lubricant), and the like, for example. The additional component can be disposed inside the polymer particles, on the surface of the polymer particles, or both thereof. The additional component may be a flowability improver (lubricant).

The flowability improver may contain inorganic particles. Examples of the inorganic particles include silica particles such as amorphous silica.

The shape of the water-absorbent resin particles B is not particularly limited, and may be substantially spherical, crushed, or granular, or may be a shape in which primary particles having these shapes are aggregated, for example.

The median particle diameter of the water-absorbent resin particles B may be 100 to 800 $\mu$m, 150 to 700 $\mu$m, 200 to 600 $\mu$m, or 250 to 500 $\mu$m.

As the water-absorbent resin particles B, one type may be used alone, or a plurality of types may be used in combination.

The water-absorbent resin particles used for the absorber may further include water-absorbent resin particles that do not correspond to any of the water-absorbent resin particles A and the water-absorbent resin particles B.

[Absorber core]

The absorber core may be composed of only the water-absorbent resin particles, or may contain the fibrous material in addition to the water-absorbent resin particles. The absorber core may be a mixture containing the water-absorbent resin particles and the fibrous material, for example. As the structure of the absorber core, a structure in which the water-absorbent resin particles and the fibrous material are uniformly mixed may be adopted. In the present specification, the fibrous material has an amorphous shape as a whole, and does not include those, in which a fibrous material is formed into a sheet shape, such as non-woven fabrics, for example.

Examples of the fibrous material include finely pulverized wood pulp; cotton; cotton linter; rayon; cellulosic fibers such as cellulose acetate; synthetic fibers such as polyamide, polyester, and polyolefin; and a mixture of these fibers. The fibrous material may be used alone, or may be used in combination of two or more types. As the fibrous material, hydrophilic fibers can be used.

An adhesive binder may be blended in the absorber core to enhance the morphological retention before and during use of the absorber core. Examples of the adhesive binder include thermal bonding synthetic fibers, hot melt adhesives, and adhesive emulsions.

Examples of the thermal bonding synthetic fibers include a total fusion type binder such as polyethylene, polypropylene, and an ethylene-propylene copolymer; and a non-total fusion type binder made of a side-by-side or core-sheath structure of polypropylene and polyethylene. In the above-mentioned non-total fusion type binder, only the polyethylene portion can be thermal-bonded.

Examples of the hot melt adhesives include a mixture of a base polymer, such as an ethylene-vinyl acetate copolymer, a styreneisoprene-styrene block copolymer, a styrene-butadiene-styrene block copolymer, a styrene-ethylene-butylene-styrene block copolymer, a styrene-ethylene-propylene-styrene block copolymer, and amorphous polypropylene, with a tackifier, a plasticizer, an antioxidant, or the like.

Examples of the adhesive emulsions include a polymerization product of at least one monomer selected from the group consisting of methyl methacrylate, styrene, acrylonitrile, 2-ethylhexyl acrylate, butyl acrylate, butadiene, ethylene, and vinyl acetate. These adhesive binders may be used alone, or may be used in combination of two or more types.

The area of the absorber core when seen in a plan view may be 300 cm$^2$ or more, or may be 330 cm$^2$ or more, 350 cm$^2$ or more, 380 cm$^2$ or more, 400 cm$^2$ or more, 430 cm$^2$ or more, 450 cm$^2$ or more, or 470 cm$^2$ or more. The area of the absorber core may be 550 cm$^2$ or less, 530 cm$^2$ or less, 500 cm$^2$ or less, or 490 cm$^2$ or less.

The shape of the absorber core may be a sheet shape, for example. The thickness of the absorber core (for example, the thickness of the sheet-shaped absorber core) may be 0.1 to 20 mm, or 0.3 to 15 mm.

[Absorber]

The absorber has the absorber core. The absorber may have core wraps for retaining the shape of the absorber core, or may consist of only the absorber core. By using the core wraps, the shape retainability of the absorber is maintained, which makes it possible to prevent the water-absorbent resin particles and the like constituting the absorber from falling off and flowing. Examples of the core wraps include non-woven fabrics, woven fabrics, tissues, synthetic resin films having liquid permeation holes, and net-like sheets having a mesh, and from the viewpoint of economic efficiency, tissues obtained by wet-molding pulverized pulp are preferably used.

The content of the water-absorbent resin particles in the absorber may be 5% to 100% by mass with respect to the total of the water-absorbent resin particles and the fibrous material. With respect to the total of the water-absorbent resin particles and the fibrous material, the content of the water-absorbent resin particles in the absorber may be 10% by mass or more, 20% by mass or more, 30% by mass or more, 40% by mass or more, 50% by mass or more, 60% by mass or more, 80% by mass or more, 90% by mass or more, or 95% by mass or more, and may be 98% by mass or less, 95% by mass or less, 90% by mass or less, 80% by mass or less, 70% by mass or less, 60% by mass or less, 50% by mass or less, 40% by mass or less, 30% by mass or less, 20% by mass or less, or 10% by mass or less.

The absorber may further contain an inorganic powder (for example, amorphous silica), a deodorant, an antibacterial agent, a dye, a pigment, a fragrance, a sticking agent, or the like. These additives can impart various functions to the absorber. When the water-absorbent resin particles include inorganic particles, the absorber may contain an inorganic powder in addition to the inorganic particles in the water-absorbent resin particles. Examples of the inorganic powder include silicon dioxide, zeolite, kaolin, and clay.

The shape of the absorber may be a sheet shape, for example. The thickness of the absorber (for example, the thickness of the sheet-shaped absorber) may be 0.1 to 20 mm, or 0.3 to 15 mm.

The planar shape of the absorber is appropriately determined according to the use or according to the shape of an absorbent article, and examples thereof include a substantially rectangular shape, an elliptical shape, an hourglass shape, and a Hagoita (Japanese battledore racket) shape. In addition, the internal structure of the absorber is also appropriately determined according to the purpose. Examples thereof include, in addition to being consisting of a single absorber, a combination of a plurality of absorbers (divided on a plane, divided in a vertical direction, and the like), and applying a quantitative distribution gradient (uniform distribution, quantitative distribution according to a liquid injection part, and the like) of the water-absorbent resin particles and the other components to the inside the absorber. In addition, the absorber may have a liquid flow path. The liquid flow path may be provided by subjecting the absorber to embossing processing, for example.

The area of the upper surface (the surface on the side where a liquid to be absorbed enters) of the absorber may be 300 $cm^2$ or more, or may be 330 $cm^2$ or more, 350 $cm^2$ or more, 380 $cm^2$ or more, 400 $cm^2$ or more, 430 $cm^2$ or more, 450 $cm^2$ or more, or 470 $cm^2$ or more. The area of the upper surface of the absorber may be 550 $cm^2$ or less, 530 $cm^2$ or less, 500 $cm^2$ or less, or 490 $cm^2$ or less.

In the absorber, the absorber core may be adhered to the core wraps by the adhesive binder. In particular, when the absorber core does not contain the fibrous material, it is suitable that the absorber core and the core wraps are adhered to each other. Examples of the adhesive binders include thermal bonding synthetic fibers, hot melt adhesives, and adhesive emulsions. The adhesive binder may be used alone, or may be used in combination of two or more types.

[Absorbent article]

The absorber of the present embodiment is suitable as a constitution of an absorbent article. In other words, the absorbent article may include the absorber of the present embodiment. Examples of other constituent members of the absorbent article include a liquid permeable sheet disposed on the outermost part at the side where a liquid to be absorbed enters; and a liquid impermeable sheet disposed on the outermost part at the opposite side to the side where a liquid to be absorbed enters. Examples of the absorbent article include diapers (for example, disposable diapers), toilet training pants, incontinence pads, sanitary materials (sanitary napkins, tampons, and the like), sweat pads, pet sheets, portable toilet members, and animal excrement treatment materials. The absorbent article may be disposable.

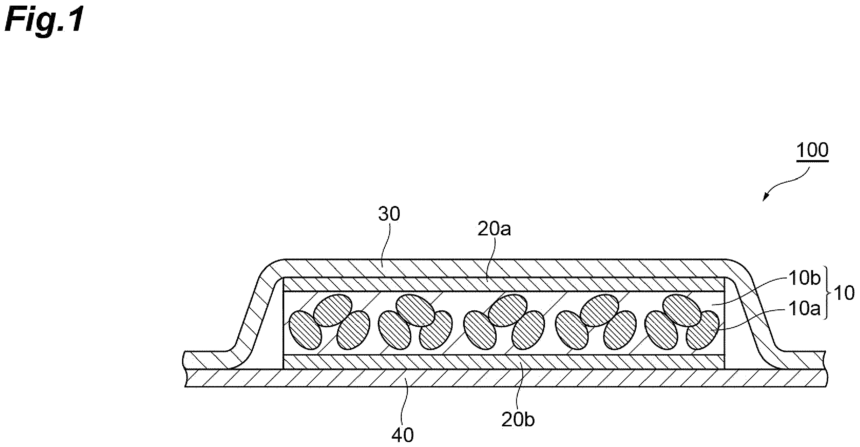

Fig. 1 is a cross-sectional view showing an example of the absorbent article. An absorbent article 100 shown in Fig. 1 has an absorber core 10, core wrap sheets 20a and 20b, a liquid permeable sheet 30, and a liquid impermeable sheet 40. In the absorbent article 100, the liquid impermeable sheet 40, the core wrap sheet 20b, the absorber core 10, the core wrap sheet 20a, and the liquid permeable sheet 30 are laminated in this order. The absorber core 10, the core wrap sheet 20a, and the core wrap sheet 20b constitute an absorber. In Fig. 1, there is a portion shown so that there is a gap between the members, but the members may be in close contact with each other without the gap.

The absorber core 10 has water-absorbent resin particles 10a and a fiber layer 10b containing a fibrous material. The water-absorbent resin particles 10a are dispersed in the fiber layer 10b. The water-absorbent resin particles 10a are the water-absorbent resin particles A, the water-absorbent resin particles B, other water-absorbent resin particles, or a mixture of these.

The core wrap sheet 20a is disposed on one surface side of the absorber core 10 (upper side of the absorber core 10 in Fig. 1) in the state of being in contact with the absorber core 10. The core wrap sheet 20b is disposed on the other surface side of the absorber core 10 (lower side of the absorber core 10 in Fig. 1) in the state of being in contact with the absorber core 10. The absorber core 10 is disposed between the core wrap sheet 20a and the core wrap sheet 20b. The core wrap sheet 20a and the core wrap sheet 20b each have a main surface having the same size as that of the absorber core 10, for example.

The liquid permeable sheet 30 is disposed on the outermost part at the side where a liquid to be absorbed enters. The liquid permeable sheet 30 is disposed on the core wrap sheet 20a in the state of being in contact with the core wrap sheet 20a. The liquid permeable sheet 30 may be a sheet formed of resins or fibers usually used in the technical field. From the viewpoint of liquid permeability, flexibility, and strength when used in the absorbent article, for example, the liquid permeable sheet 30 may contain synthetic resins such as polyolefins such as polyethylene (PE) and polypropylene (PP), polyesters such as polyethylene terephthalate (PET), polytrimethylene terephthalate (PTT), and polyethylene naphthalate (PEN), polyamides such as nylon, and rayon; or synthetic fibers containing these synthetic resins, or the liquid permeable sheet 30 may be natural fibers including cotton, silk, hemp, or pulp (cellulose). The liquid permeable sheet 30 may contain synthetic fibers from the viewpoint of increasing the strength of the liquid permeable sheet 30. In particular, the synthetic fibers may be polyolefin fibers, polyester fibers, or a combination thereof. These materials may be used alone or may be used in combination of two or more types of materials.

The liquid permeable sheet 30 may be a non-woven fabric, a porous sheet, or a combination thereof. The non-woven fabric is a sheet in which fibers are entwined instead of being woven. The non-woven fabric may be a non-woven fabric (short-fiber non-woven fabric) composed of short fibers (that is, staples), or may be a non-woven fabric (long-fiber non-woven fabric) composed of long fibers (that is, filaments). In general, staples may have a fiber length of several hundred millimeters or shorter, but there is no limitation.

The liquid permeable sheet 30 may be a thermal bonded non-woven fabric, an air through non-woven fabric, a resin bonded non-woven fabric, a spunbond non-woven fabric, a melt-blown non-woven fabric, an airlaid non-woven fabric, a spunlace non-woven fabric, a point-bonded non-woven fabric, or a laminate of two or more types of non-woven fabrics selected from these non-woven fabrics. These non-woven fabrics may be those formed of the above-mentioned synthetic fibers or natural fibers, for example. For example, the laminate of two or more types of non-woven fabrics may be a spunbond/melt-blown/spunbond non-woven fabric that is a composite non-woven fabric having a spunbond non-woven fabric, a melt-blown non-woven fabric, and a spunbond non-woven fabric, which are laminated in this order. The liquid permeable sheet 30 may be a thermal bonded non-woven fabric, an air through non-woven fabric, a spunbond non-woven fabric, or a spunbond/melt-blown/spunbond non-woven fabric, from the viewpoint of preventing liquid leakage.

It is desirable that a non-woven fabric used as the liquid permeable sheet 30 have appropriate hydrophilicity to improve liquid absorption performance of the absorbent article. From this viewpoint, the liquid permeable sheet 30 may be a non-woven fabric having a hydrophilicity of 5 to 200 measured according to a measurement method of Pulp and Paper Test Method No. 68 (2000) by the Japan Technical Association of Pulp and Paper Industry. The above-mentioned hydrophilicity of the non-woven fabric may be 10 to 150. For details of Pulp and Paper Test Method No. 68, WO2011/086843 can be referred to, for example.

The hydrophilic non-woven fabric as mentioned above may be formed of fibers, such as rayon fibers, showing appropriate hydrophilicity, or may be formed of fibers obtained by hydrophilizing hydrophobic chemical fibers such as polyolefin fibers and polyester fibers. Examples of a method of obtaining a non-woven fabric containing hydrophobic chemical fibers that have been hydrophilized include a method of obtaining a non-woven fabric by a spunbond technique using one obtained by mixing a hydrophilizing agent with hydrophobic chemical fibers, a method of using a hydrophilizing agent when producing a spunbond non-woven fabric with hydrophobic chemical fibers, and a method of impregnating a spunbond non-woven fabric obtained using hydrophobic chemical fibers with a hydrophilizing agent. As the hydrophilizing agent, anionic surfactants such as aliphatic sulfonic acid salts and higher alcohol sulfuric acid ester salts; cationic surfactants such as quaternary ammonium salts; nonionic surfactants such as polyethylene glycol fatty acid esters, polyglycerin fatty acid esters, and sorbitan fatty acid esters; silicone surfactants such as polyoxyalkylene-modified silicone; stain release agents formed of polyester-based, polyamide-based, acrylic-based, or urethane-based resin; and the like are used.

From the viewpoint of imparting favorable liquid permeability, flexibility, strength, and cushioning properties to the absorbent article, and from the viewpoint of accelerating a liquid permeation rate of the absorbent article, the liquid permeable sheet 30 may be a non-woven fabric that is moderately bulky and has a large fabric weight per unit area. The fabric weight per unit area of the non-woven fabric used for the liquid permeable sheet 30 may be 5 to 200 g/m$^2$, may be 8 to 150 g/m$^2$, or may be 10 to 100 g/m$^2$. The thickness of the non-woven fabric used for the liquid permeable sheet 30 may be 20 to 1,400 $\mu$m, may be 50 to 1,200 $\mu$m, or may be 80 to 1,000 $\mu$m.

In addition, the surface of the liquid permeable sheet 30 may be subjected to embossing processing or perforation processing to improve the diffuseness of liquids. The embossing processing or perforation processing can be performed by a known method. Furthermore, a skin toner, a moisturizing agent, an antioxidant, an anti-inflammatory agent, a pH adjuster, or the like may be blended in the liquid permeable sheet 30 to reduce irritation to the skin. The shape of the liquid permeable sheet 30 depends on the shapes of the absorber and the absorbent article, but may be a shape in which the absorber core 10 is covered such that liquid leakage does not occur.

The liquid impermeable sheet 40 is disposed on the opposite side to the liquid permeable sheet 30, with the absorber sandwiched therebetween, in the absorbent article 100. The liquid impermeable sheet 40 is disposed below the core wrap sheet 20b in the state of being in contact with the core wrap sheet 20b. For example, the liquid impermeable sheet 40 has a main surface wider than the main surface of the absorber core 10, and outer edges of the liquid impermeable sheet 40 extend around the absorber core 10 and the core wrap sheet 20b. The liquid impermeable sheet 40 prevents a liquid absorbed by the absorber core 10 from leaking to the outside from the liquid impermeable sheet 40 side.

Examples of the liquid impermeable sheet 40 include sheets made of synthetic resins such as polyethylene, polypropylene, and polyvinyl chloride; sheets made of non-woven fabric such as a spunbond/melt-blown/spunbond (SMS) non-woven fabric in which a water-resistant melt-blown non-woven fabric is sandwiched between high-strength spunbond non-woven fabrics; and sheets made of a composite material of these synthetic resins and a non-woven fabric (for example, a spunbond non-woven fabric and a spunlace non-woven fabric). The liquid impermeable sheet 40 may have breathability from the viewpoint that dampness when worn is reduced, which makes it possible to reduce discomfort to a wearer. As the liquid impermeable sheet 40, a sheet made of a synthetic resin mainly made of a low-density polyethylene (LDPE) resin can be used. For example, the liquid impermeable sheet 40 may be a sheet made of a synthetic resin and having a fabric weight per unit area of 10 to 50 $g/m^2$ from the viewpoint of ensuring flexibility so as not to impair a sense of wearing the absorbent article. Furthermore, to impart breathability to the liquid impermeable sheet 40, for example, a filler may be blended in a resin sheet, or the liquid impermeable sheet 40 may be subjected to embossing processing. As the filler, calcium carbonate or the like is used.

For example, the liquid permeable sheet 30 and the liquid impermeable sheet 40 have a main surface wider than the main surface of the absorber core 10, and the outer edges of the liquid permeable sheet 30 and the liquid impermeable sheet 40 extend around the absorber core 10 and the core wrap sheets 20a and 20b.

The magnitude relationship between the absorber core 10, the core wrap sheets 20a and 20b, the liquid permeable sheet 30, and the liquid impermeable sheet 40 is not particularly limited, and is appropriately adjusted according to the use of the absorbent article or the like. In addition, a method of retaining the shape of the absorber core 10 using the core wrap sheets 20a and 20b is not particularly limited, and the absorber core may be wrapped by a plurality of core wrap sheets as shown in Fig. 1, or the absorber core may be wrapped by one core wrap sheet.

For example, the absorbent article 100 can be produced by a method including disposing the absorber core 10 between the core wrap sheets 20a and 20b, and disposing them between the liquid permeable sheet 30 and the liquid impermeable sheet 40. A laminate, in which the liquid impermeable sheet 40, the core wrap sheet 20b, the absorber core 10, the core wrap sheet 20a, and the liquid permeable sheet 30 are laminated in this order, is pressurized if necessary.

The shape of the absorbent article 100 is appropriately determined according to the use, and when the absorbent article is a urine pad or a sanitary napkin, examples thereof include a substantially rectangular shape, an elliptical shape, an hourglass shape, and a Hagoita (Japanese battledore racket) shape.

In the absorbent article, in addition to the liquid permeable sheet, the absorber, the liquid impermeable sheet, and the core wrap mentioned above, a member may be appropriately present depending on the use and functions. Examples thereof include a liquid acquisition diffusion sheet to be described later.

(Liquid acquisition diffusion sheet)

Although not shown in Fig. 1, the absorbent article may have the liquid acquisition diffusion sheet. The liquid acquisition diffusion sheet may be disposed on the lower surface of the liquid permeable sheet 30, for example. Thus, a liquid permeated through the liquid permeable sheet 30 can be quickly moved to the absorber core 10 side, or re-wet can be further reduced. For the adhesion between the liquid acquisition diffusion sheet and the liquid permeable sheet 30, a hot melt adhesive may be used, or alternatively, hot embossing or ultrasonic welding may be used. As the liquid acquisition diffusion sheet, a resin film having a large number of permeation holes can also be used, in addition to using a non-woven fabric. As the non-woven fabric, the same materials as those described in the section of the liquid permeable sheet 30 can be used, but one having higher hydrophilicity than the liquid permeable sheet 30 or one having a higher fiber density is preferable because it is better in characteristics of liquid transfer toward the absorber.

The liquid acquisition diffusion sheet is usually disposed at the central portion in a width shorter than that of the absorber core 10, but may be disposed over the entire width. The length in the front-back direction of the liquid acquisition diffusion sheet may be substantially the same as the total length of the absorbent article, may be substantially the same as the total length of the absorber core 10, or may be a length within a range obtained by assuming a portion to which a liquid is injected.

(Outer cover non-woven fabric)

Furthermore, the outer cover non-woven fabric may be disposed on the outer side of the liquid impermeable sheet 40. The outer cover non-woven fabric can be adhered to the liquid impermeable sheet 40 using an adhesive, for example. The outer cover non-woven fabric may be formed of one or more layers, and may be a soft material. To be able to appeal to consumer's purchase desires or for other reasons, the outer cover non-woven fabric may be imparted with a soft touch feeling, may have a printed pattern, or may be formed in the forms of a plurality of bond portions, embossing processing, or three dimension.

(Leg gather (side leak guard))

The absorbent article of the present embodiment may have a leg gather (side leak guard), which has an elastic member having stretchability, which is disposed at the outer side than both end parts in the width direction of the absorber core 10, and which is installed substantially parallel to the longitudinal direction of the absorber core 10.

The length of the leg gather is set to be about equal to or greater than that of the wearer's inguinal region. The extension rate of the leg gather is appropriately set from the viewpoint of preventing the leakage of a discharged liquid and reducing a tight feeling when worn for a long period of time.

(Front/back gathers (guards))

The absorbent article of the present embodiment may have front/back gathers (guards) which are disposed in the vicinity of both end parts in the longitudinal direction of the absorbent article and have elastic members that stretch and contract in the width direction.

The absorbent article has front/back gathers capable of standing upwards at the upper part of side edges of the absorber core 10 in the width direction. In other words, at each of both sides of the absorbent article in the longitudinal direction, sheet members of the front/back gathers having the gather elastic members are disposed to form the front/back gathers.

cross-link Examples thereof include liquid impermeable or water repellent porous sheets, liquid impermeable or water repellent non-woven fabrics, and a laminate of the above-mentioned porous sheet and the above-mentioned non-woven fabric. Examples of the non-woven fabrics include thermal bonded non-woven fabrics, spunbond non-woven fabrics, melt-blown non-woven fabrics, spunlace non-woven fabrics, and spunbond/melt-blown/spunbond non-woven fabrics. The fabric weight per unit area of the above-mentioned member may be 5 to 100 $g/m^2$, may be 8 to 70 $g/m^2$, or may be 10 to 40 $g/m^2$.

**Examples**

Hereinafter, the present invention will be more specifically described with reference to examples. However, the present invention is not limited to these examples.

<Production of particles for evaluation>

[Production Example 1]

A round-bottomed cylindrical separable flask with an inner diameter of 11 cm and a volume of 2 L equipped with a reflux cooling device, a dropping funnel, a nitrogen gas introduction tube, and a stirrer (a stirrer blade having two stages of four inclined paddle blades having a blade diameter of 5 cm) was prepared. To this separable flask, 293 g of n-heptane (hydrocarbon dispersion medium) and 0.736 g of a maleic anhydride-modified ethylene-propylene copolymer (polymeric dispersant, Mitsui Chemicals, Inc., Hi-Wax 1105A) were added to obtain a mixture. The dispersant was dissolved by heating to 80°C while stirring this mixture at a rotation speed of 300 rpm. Thereafter, the mixture was cooled to 55°C.

Subsequently, 92.0 g (acrylic acid: 1.03 mol) of an aqueous solution of 80.5% by mass acrylic acid was put into a triangular flask having a volume of 500 mL. Subsequently, while cooling from the outside, 102.2 g of an aqueous solution of 30% by mass sodium hydroxide was added dropwise to neutralize 75 mol% of acrylic acid. Thereafter, 0.092 g of hydroxyethyl cellulose (thickener, Sumitomo Seika Chemicals Co., Ltd., HEC AW-1 5F), 0.0736 g (0.272 mmol) of potassium persulfate (water-soluble radical polymerization initiator), 0.0101 g (0.0580 mmol) of ethylene glycol diglycidyl ether (internal crosslinking agent), and 32.85 g of ion-exchanged water were added and then dissolved to prepare a first stage monomer aqueous solution.

The above-mentioned first stage monomer aqueous solution was added to the above-mentioned separable flask, and thereafter stirring was performed for 10 minutes. 0.736 g of sucrose stearic acid ester (surfactant, manufactured by Mitsubishi-Chemical Foods Corporation, Ryoto Sugar Ester S-370, HLB: 3) was heat-dissolved in 6.62 g of n-heptane to obtain a surfactant solution. 7.356 g of the obtained surfactant solution was added to the separable flask to obtain a reaction solution. Then, the inside of the separable flask system was sufficiently replaced with nitrogen while stirring the reaction solution at the rotation speed of 550 rpm. Thereafter, the separable flask was immersed in a water bath at 70°C to raise the temperature of the reaction solution, and first stage polymerization was performed for 10 minutes to obtain a first stage reaction mixture.

Subsequently, 128.8 g (acrylic acid: 1.44 mol) of an aqueous solution of 80.5% by mass acrylic acid was put into another triangular flask having a volume of 500 mL. Subsequently, while cooling from the outside, 143.1 g of an aqueous solution of 30% by mass sodium hydroxide was added dropwise to neutralize 75 mol% of acrylic acid. Thereafter, 0.1030 g (0.3810 mmol) of potassium persulfate, 0.0116 g (0.0666 mmol) of ethylene glycol diglycidyl ether (internal crosslinking agent), and 0.63 g of ion-exchanged water were added and then dissolved to prepare a second stage monomer aqueous solution.

Cooling to 25°C was performed while stirring the first stage reaction mixture at a rotation speed of 1,000 rpm, and then the total amount of the second stage monomer aqueous solution was added to the first stage reaction mixture to obtain a reaction solution. The inside of the system was sufficiently replaced with nitrogen while stirring the reaction solution. Thereafter, the separable flask was immersed in a water bath at 70°C to raise the temperature of the reaction solution, and second stage polymerization was performed for 5 minutes to obtain a second stage reaction mixture (polymer particles before surface crosslinking).

After the second stage polymerization, the temperature of the second stage reaction mixture was raised in an oil bath at 125°C, and 254 g of water was extracted to the outside of the system while refluxing n-heptane by azeotropic distillation of n-heptane and water. Subsequently, 0.0884 g (0.5075 mmol) of ethylene glycol diglycidyl ether was added as a surface crosslinking agent, and by maintaining at 83°C for 2 hours thereafter, a dispersion liquid of the polymer particles after surface crosslinking was obtained.

Thereafter, the temperature of the above-mentioned dispersion liquid of the polymer particles after surface crosslinking was raised in an oil bath at 125°C, and drying was performed by evaporating n-heptane to obtain a dried product. This dried product was passed through a sieve having an opening of 850 μm to obtain 232.8 g of water-absorbent resin particles (a) in the form of aggregated spherical particles and in an uncoated state.

Thereafter, 200 g of the above-mentioned water-absorbent resin particles (a), and 0.4 g of Tokusil (Oriental Silicas Corporation, product number: NP-S) as a hydrophilic silica were put in a SUS bottle and mixed for 30 minutes with a cross rotary mixer to obtain water-absorbent resin particles (1). The water-absorbent resin particles (1) correspond to the water-absorbent resin particles B.

[Production Example 2]

Until adding the hydrophilic silica, Production Example 1 was repeated, and the collected water-absorbent resin particles (a) were classified with a comb having an opening of 250 μm to obtain 500 g or more of water-absorbent resin particles (b) having a particle diameter of 250 to 850 μm.

(Coating operation of water-absorbent resin particles (b))

25.0 g of polyvinyl chloride (FUJIFILM Wako Pure Chemical Corporation) as a coating material and 475.0 g of tetrahydrofuran as a solvent were added to a polybeaker having an internal volume of 1 L and mixed to prepare a coating liquid A.

500.0 g of the water-absorbent resin particles (b) was injected to a container of a fluid bed granulator, and hot air at 40°C was blown from the lower part of the container. Subsequently, while drying 500.0 g of the coating liquid A, it was sprayed on the water-absorbent resin particles (b) wound up by blowing. After spraying the coating liquid, drying was performed at 40°C for 30 minutes.

After drying, 503.3 g of coated resin particles (2) was obtained. The coated resin particles (2) correspond to the water-absorbent resin particles A.

[Production Example 3]

200.0 g of a 25% aqueous dispersion emulsion of an ethylene-sodium acrylate copolymer (Sumitomo Seika Chemicals Co., Ltd., ZAIKTHENE N) and 5.0 g of polyethylene glycol (Tokyo Chemical Industry Co., Ltd., PEG 6000) were diluted with 295.0 g of ion-exchanged water to prepare a coating liquid B. 505.9 g of coated resin particles was obtained by performing coating in the same manner as in Production Example 2 except that the coating liquid B was used instead of the coating liquid A, and that the drying condition after blowing hot air from the lower part of the container and spraying the coating liquid was changed to 50°C.

50.0 g of the obtained coated resin particles was spread on a metal vat having a length of 26 cm and a width of 20 cm, and was covered with aluminum foil. The aluminum foil was perforated, and the coated resin particles were heated for 60 minutes by a hot-air dryer (ADVANTEC, FV-320) set at 80°C to obtain 50.0 g of coated resin particles (3). The coated resin particles (3) correspond to the water-absorbent resin particles A.

[Production Example 4]

25.0 g of polymethyl methacrylate (FUJIFILM Wako Pure Chemical Corporation) as a coating material and 475 g of acetone as a solvent were added to a polybeaker having an internal volume of 1 L and mixed to prepare a coating liquid C. 502.4 g of coated resin particles (4) was obtained by performing coating in the same manner as in Production Example 2 except that the coating liquid C was used instead of the coating liquid A.

[Production Example 5]

234.5 g of water-absorbent resin particles (5) in an uncoated state was obtained in the same manner as in Production Example 1 except that, in obtaining the water-absorbent resin particles in the uncoated state, 0.00534 g (0.0306 mmol) of ethylene glycol diglycidyl ether (internal crosslinking agent) was added when preparing a first stage monomer aqueous solution, and the addition amount of hydrophilic silica was 1.0 g. The water-absorbent resin particles (5) correspond to the water-absorbent resin particles B.

[Production Example 6]

550.0 g of water-absorbent resin particles (6) in an uncoated state, which were collected from a commercially available diaper sold in China (manufactured by The Procter & Gamble Company, trade name: Pampers, Refreshing, NB size), was obtained. The water-absorbent resin particles (6) correspond to the water-absorbent resin particles B.

[Production Example 7]

50.0 g of coated resin particles (7) was obtained by performing coating in the same manner as in Production Example 3 except that the coating was performed using the uncoated water-absorbent resin (5) produced in Production Example 5. The coated resin particles (7) correspond to the water-absorbent resin particles A.

[Production Example 8]

50.0 g of coated resin particles (8) was obtained by performing coating in the same manner as in Production Example 3 except that the coating was performed using the uncoated water-absorbent resin (6) collected in Production Example 6. The coated resin particles (8) correspond to the water-absorbent resin particles A.

[Production Example 9]

50.0 g of coated resin particles (9) was obtained by producing uncoated water-absorbent resin particles in the same manner as in Production Example 1 and thereafter performing coating in the same manner as in Production Example 3 except that, in obtaining the water-absorbent resin particles in the uncoated state, after the second stage polymerization, the temperature of a second stage reaction mixture was raised in an oil bath at 125°C, and 272 g of water was extracted to the outside of the system while refluxing n-heptane by azeotropic distillation of n-heptane and water; and hydrophilic silica was not mixed. The coated resin particles (9) correspond to the water-absorbent resin particles A.

<Particle evaluation>

The water-absorbent resin particles or the coated resin particles obtained in Production Examples 1 to 9 were evaluated as follows.

[Water absorption rate: lock-up height]

0.200 g of the particles was weighed exactly and spread on the bottom of an acrylic cylinder with an inner diameter of 2.0 cm and a depth of 8.0 cm to measure a height $H_0$ of a particle layer. Thereafter, 20 g of physiological saline was weighed and poured into the acrylic cylinder from its upper part. The measurement was started when the total amount of the physiological saline was put, and a height $H_2$ of the particle layer was read after 2 minutes to calculate the water absorption rate from the following formula. The results are shown in Table 1.

$$\text{Water absorption rate [cm]} = H_2 - H_0$$

[Water retention capacity]

After adding 500 g of physiological saline to a 500 mL polyethylene beaker, 2.00 g of the particles was added little by little while rotating a stirring bar at a rotation speed of 600 rpm using a stirrer. After addition of the total amount of the particles was completed, stirring was performed for 30 minutes. Subsequently, after transferring the mixture into a cotton bag, the upper part of the cotton bag was closed with a rubber band. Subsequently, centrifugation (167 G) was performed for 1 minute using a centrifuge. A mass $W_A$ was measured after dehydration. The same operation was performed without putting the particles in the cotton bag to measure a mass $W_B$ of the empty cotton bag. The water retention capacity (25°C) of the physiological saline was calculated from the following formula.

The results are shown in Table 1.

$$\text{Water retention capacity [g/g]} = (W_A - W_B)/\text{mass of particles}$$

[Table 1]

| | | Water retention capacity [g/g] | 2-Minute value of water absorption rate [cm] |
|---|---|---|---|
| Production Example 1 | Water-absorbent resin particles (1) | 42 | 4.6 |
| Production Example 2 | Coated resin particles (2) | 29 | 0.3 |
| Production Example 3 | Coated resin particles (3) | 38 | 0.4 |
| Production Example 4 | Coated resin particles (4) | 39 | 2.9 |
| Production Example 5 | Water-absorbent resin particles (5) | 48 | 4.6 |
| Production Example 6 | Water-absorbent resin particles (6) | 41 | 2.5 |
| Production Example 7 | Coated resin particles (7) | 45 | 0.3 |
| Production Example 8 | Coated resin particles (8) | 38 | 0.3 |
| Production Example 9 | Coated resin particles (9) | 52 | 0.3 |

<Production of absorber>

[Production Example A]

A total of 12.0 g of the water-absorbent resin particles (one type or a plurality of types among the water-absorbent resin particles (1) and the coated resin particles (2) to (4), the same applies hereinafter) and 108 g of crushed pulp (Rayfloc manufactured by Rayonier Inc.) were used and uniformly mixed by air papermaking (suction pressure 0.75 ± 0.05 MPa, conveyor speed 4.8 sec/cm) with an air flow type mixer (manufactured by Otec Co., Ltd., Padformer), thereby producing an absorber core with a size of 40 cm × 12 cm. Subsequently, in a state of sandwiching the upper part and the lower part of the absorber core between two sheets of tissue paper having the same area as that of the absorber core and having a basis weight of 16 g/m², a load of 424 kPa was applied to the entirety for 30 seconds to press it, thereby producing an absorber. The content of the water-absorbent resin particles in this absorber (content of the water-absorbent resin particles in the total amount of the water-absorbent resin particles and the crushed pulp (fibrous material) in the absorber, the same applies hereinafter) was 10% by mass. Furthermore, an air-through type polyethylene porous liquid permeable sheet having a basis weight of 22 g/m$^2$ and having the same area as that of the absorber was disposed on the upper surface of the absorber to obtain an absorbent article.

[Production Example B]

An absorber and an absorbent article were produced in the same manner as in Production Example A except that the amount of the crushed pulp was changed to 28 g, and the load applied to the entirety was changed to 141 kPa. The content of the water-absorbent resin particles in the absorber was 30% by mass.

[Production Example C]

An absorber and an absorbent article were produced in the same manner as in Production Example B except that the amount of the crushed pulp was changed to 8 g. The content of the water-absorbent resin particles in the absorber was 60% by mass.

[Production Example D]

An airlaid non-woven fabric (China Silk (Shanghai) New Material Technology Co., Ltd., MB0401-T1) having a fabric weight per unit area of 38 g/m$^2$ was cut into two pieces having a size of 14 cm × 42 cm which were respectively defined as airlaid non-woven fabrics 1 and 2.
A total amount of 0.2 g of a hot melt adhesive (Henkel Japan Ltd., ME-765E) was applied to the airlaid non-woven fabric 2 in 14 straight lines at intervals of 10 mm with a hot melt coating machine (HALLYS Corporation, pump: Marshal 150, table: XA-DT, tank set temperature: 150°C, set temperature in hose: 165°C, gun head set temperature: 170°C). The application pattern of the adhesive was a spiral stripe. When coating, four outer peripheral sides (width 1 cm) of the airlaid non-woven fabric 2 were covered with a cover so that the adhesive was not applied thereto.
12 g of the water-absorbent resin particles was uniformly scattered over a range of 12 cm × 40 cm at the center of the surface of the airlaid non-woven fabric 2 to which the hot melt was adhered.
0.2 g of the hot melt was applied to the airlaid non-woven fabric 1 by the same operation as mentioned above but without covering the outer periphery thereof. Both ends of the airlaid non-woven fabric 2 and the airlaid non-woven fabric 1 were aligned such that the adhesive-applied surfaces were on the inner side, these fabrics were sandwiched between release papers, and pressed under the conditions of 110°C and 0.1 MPa to attach using a laminating machine (HASHIMA CO., LTD., Straight Linear Fussing Press, model HP-600LFS), thereby producing an absorber. The range in which the water-absorbent resin particles were scattered in the absorber was 12 cm × 40 cm. An air-through type polyethylene porous liquid permeable sheet having a basis weight of 22 g/m$^2$ and having the same area as that of the absorber was further disposed on the upper surface of the absorber, thereby obtaining an absorbent article. The content of the water-absorbent resin particles in the absorber was 100% by mass.

[Example 1]

An absorbent article was produced by the production method of Production Example A using 9.6 g of the water-absorbent resin particles (1) and 2.4 g of the coated resin particles (2) as the water-absorbent resin particles.

[Comparative Example 1]

An absorbent article was produced by the production method of Production Example A using 3.6 g of the water-absorbent resin particles (1) and 8.4 g of the coated resin particles (2) as the water-absorbent resin particles.

[Comparative Example 2]

An absorbent article was produced by the production method of Production Example B using 12 g of the water-absorbent resin particles (1) as the water-absorbent resin particles.

[Example 2]

An absorbent article was produced by the production method of Production Example B using 10.8 g of the water-absorbent resin particles (1) and 1.2 g of the coated resin particles (2) as the water-absorbent resin particles.

[Example 3]

An absorbent article was produced by the production method of Production Example B using 9.6 g of the water-absorbent resin particles (1) and 2.4 g of the coated resin particles (2) as the water-absorbent resin particles.

[Example 4]

An absorbent article was produced by the production method of Production Example B using 7.2 g of the water-absorbent resin particles (1) and 4.8 g of the coated resin particles (2) as the water-absorbent resin particles.

[Example 5]

An absorbent article was produced by the production method of Production Example B using 4.8 g of the water-absorbent resin particles (1) and 7.2 g of the coated resin particles (2) as the water-absorbent resin particles.

[Example 6]

An absorbent article was produced by the production method of Production Example B using 9.6 g of the water-absorbent resin particles (1) and 2.4 g of the coated resin particles (3) as the water-absorbent resin particles.

[Comparative Example 3]

An absorbent article was produced by the production method of Production Example C using 12 g of the water-absorbent resin particles (1) as the water-absorbent resin particles.

[Example 7]

An absorbent article was produced by the production method of Production Example C using 9.6 g of the water-absorbent resin particles (1) and 2.4 g of the coated resin particles (2) as the water-absorbent resin particles.

[Example 8]

An absorbent article was produced by the production method of Production Example C using 9.6 g of the water-absorbent resin particles (1) and 2.4 g of the coated resin particles (3) as the water-absorbent resin particles.

[Comparative Example 4]

An absorbent article was produced by the production method of Production Example C using 9.6 g of the water-absorbent resin particles (1) and 2.4 g of the coated resin particles (4) as the water-absorbent resin particles.

[Comparative Example 5]

An absorbent article was produced by the production method of Production Example C using 3.6 g of the water-absorbent resin particles (1) and 8.4 g of the coated resin particles (2) as the water-absorbent resin particles.

[Comparative Example 6]

An absorbent article was produced by the production method of Production Example D using 12 g of the water-absorbent resin particles (1) as the water-absorbent resin particles.

[Example 9]

An absorbent article was produced by the production method of Production Example D using 9.6 g of the water-absorbent resin particles (1) and 2.4 g of the coated resin particles (2) as the water-absorbent resin particles.

[Comparative Example 7]

An absorbent article was produced by the production method of Production Example D using 9.6 g of the water-absorbent resin particles (1) and 2.4 g of the coated resin particles (4) as the water-absorbent resin particles.

[Comparative Example 8]

An absorbent article was produced by the production method of Production Example D using 3.6 g of the water-absorbent resin particles (1) and 8.4 g of the coated resin particles (2) as the water-absorbent resin particles.

[Comparative Example 10]

An absorbent article was produced by the production method of Production Example C using 12.0 g of the water-absorbent resin particles (5) as the water-absorbent resin particles.

[Example 10]

An absorbent article was produced by the production method of Production Example C using 7.2 g of the water-absorbent resin particles (5) and 4.8 g of the coated resin particles (3) as the water-absorbent resin particles.

[Example 11]

An absorbent article was produced by the production method of Production Example C using 9.6 g of the water-absorbent resin particles (6) and 2.4 g of the coated resin particles (7) as the water-absorbent resin particles.

[Example 12]

An absorbent article was produced by the production method of Production Example C using 9.6 g of the water-absorbent resin particles (6) and 2.4 g of the coated resin particles (8) as the water-absorbent resin particles.

[Example 13]

An absorbent article was produced by the production method of Production Example C using 9.6 g of the water-absorbent resin particles (1) and 2.4 g of the coated resin particles (9) as the water-absorbent resin particles.

<Performance evaluation of absorber>

(1) Preparation of test solution

100.0 g of sodium chloride, 3.0 g of calcium chloride dihydrate, 6.0 g of magnesium chloride hexahydrate, 1% Triton (prepared by mixing 1.0 g of polyoxyethylene[10] octalphenyl ether X-100 and 99.0 g of distilled water), and 9866.0 g of distilled water were put in a container having a volume of 10 L and were completely dissolved. Furthermore, the solution was colored with a small amount of Blue No. 1 to prepare a test solution.

(2) Measurement of absorption rate and diffusion area of absorber

First, the absorbent article was placed on a horizontal table. For only in the absorbent article of Production Example D, a frame having a length of 42 cm, a width of 14 cm, and a height of 1 cm was installed around the absorbent article to prevent lateral leakage. A liquid injection cylinder (59.80 g) having an opening with an outer diameter of 3.5 cm and an inner diameter of 3 cm was placed on the center of the absorbent article, and 80 mL of the test solution was injected into the cylinder at one time. The time from the start of injection to the completion of absorption was measured as the absorption rate. Immediately after the absorption was completed, the cylinder was removed from the absorbent article.

Subsequently, 20 minutes after the start of injection of artificial urine, a transparent polyethylene bag with a zipper (Unipack (registered trademark), area 1,200 cm$^2$, weight 4.8 g) for which the area and the weight had been previously measured was placed on the absorbent article, and a mark was made by tracing the outer periphery of the blue area in which the artificial urine was diffused on the liquid permeable sheet.

30 minutes after the first artificial urine injection, the second artificial urine injection was performed in the same manner as in the first time to perform measurement of the absorption rate and diffusion range recording after 20 minutes in the same manner as in the first time. These operations were repeated twice more, and a total of four times was performed.

The absorption rates at the time of injecting artificial urine four times in total were added up, which was defined as the absorption rate of the absorber. In addition, each of transparent polyethylene bags with a zipper, for which the diffusion range after four times of artificial urine injection was recorded, was cut out according to the shape to measure masses ($W_1$, $W_2$, $W_3$, and $W_4$). The diffusion areas ($S_1$, $S_2$, $S_3$, and $S_4$) at each liquid injection were calculated from the following formula. $W_n$ and $S_n$ respectively refer to the mass of the transparent bag and the diffusion area at the time of the nth artificial urine injection. Although the diffusion range recording of the absorber was performed on a portion beyond the liquid permeable sheet of the absorbent article, there is substantially no difference between the diffusion range on the surface of the absorber and the diffusion range on the liquid permeable sheet.

$$S_n = (W_1 \times 1200)/4.8$$

Subsequently, using the diffusion areas ($S_1$, $S_2$, $S_3$, and $S_4$), the diffusion area change ratio was calculated by the following formula.

$$\text{Diffusion area change ratio} = (S_1 + S_2)/(S_3 + S_4)$$

In addition, an effective diffusion area fraction was calculated by the following formula. In the above-mentioned measurement, the area of the absorber core was 480 $cm^2$.

$$\text{Effective diffusion area fraction (\%)} = (S_4/\text{area of absorber core})$$

$$\times 100$$

(3) Leakage test

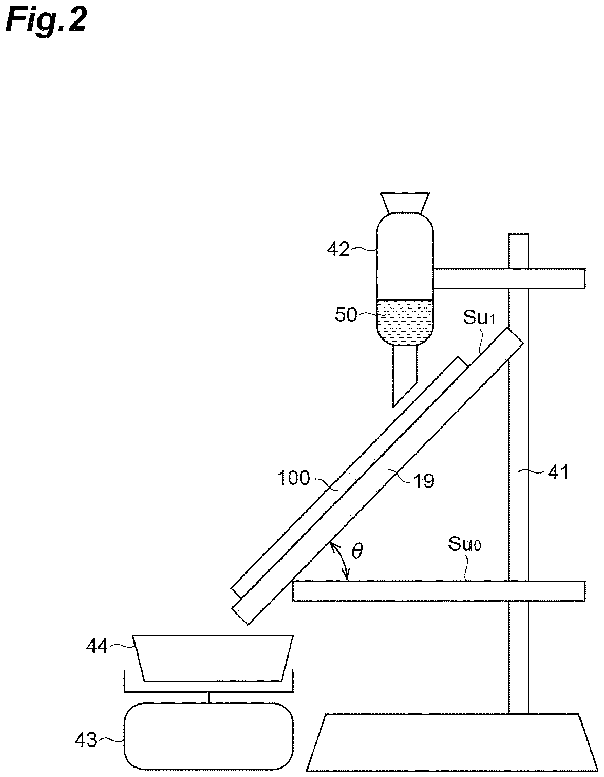

Fig. 2 is a schematic view showing a method for evaluating leakage properties of an absorbent article. A support plate 19 (herein an acrylic resin plate) having a length of 45 cm and a thickness of 0.3 cm and having a flat inclined surface $Su_1$ was fixed by a stand 41 in the state of being inclined at a predetermined angle to be described later with respect to a horizontal plane $Su_0$. A dropping funnel with a volume of 300 mL had a distal end portion with an inner diameter of about 8 mmφ and a two-way cock, and a stop of the cock was adjusted such that a liquid was injected at 8 mL/sec. A balance 43 on which a metal tray 44 was placed was installed at the lower part of the support plate 19 to receive all drops of the test solution flowing down as leaks from the end of the absorbent article, and the mass thereof was recorded with an accuracy of 0.1 g. A leakage test using such a device was performed according to the following procedure.

The absorbent article 100 for testing was attached onto the inclined surface $Su_1$ of the fixed support plate 19 in a room at a temperature of 25 ± 2°C such that the longitudinal direction of the absorbent article 100 was along the longitudinal direction of the support plate 19. The lower end of the absorbent article was not attached onto the acrylic plate in order not to intentionally stop leakage. Subsequently, a test solution 50 (artificial urine) adjusted to 25°C ± 1°C was added dropwise from a dropping funnel 42 vertically disposed above the absorbent article toward the center of the absorber in the absorbent article 100. A distance between the distal end of the dropping funnel 42 and the absorbent article was 10 ± 2 mm. The surface of the support plate 19 was smooth, and the liquid did not stay on or was not absorbed by the plate. When the test solution that was not absorbed by the absorbent article 100 leaked out from the lower part of the support plate 19, the leaked test solution was recovered in the metal tray 44 disposed below the support plate 19. A weight (g) of the recovered test solution was measured by the balance 43, and this value was recorded as an amount of leakage.

When performing the leakage test, in Production Example A, the angle of the acrylic plate with respect to the horizontal plane was set to 45° ± 2°, the injection position of the test solution 50 into the absorbent article was set to a mark portion, and the injection amount of the test solution was set to 600 g. In Production Example B, the angle of the acrylic plate with respect to the horizontal plane was set to 30° ± 2°, the injection position of the test solution 50 into the absorbent article was set to a mark portion, and the injection amount of the test solution was set to 300 g. In Production Example C, the angle of the acrylic plate with respect to the horizontal plane was set to 30° ± 2°, the injection position of the test solution 50 into the absorbent article was set to a position 5 cm above a mark portion, and the injection amount of the test solution was set to 150 g. In Production Example D, the angle of the acrylic plate with respect to the horizontal plane was set to 30° ± 2°, the injection position of the test solution 50 into the absorbent article was set to a position 5 cm above a mark portion, and the injection amount of the test solution was set to 50 g.

Table 2 shows the results of Production Example A (the content of the water-absorbent resin particles in the total amount of the water-absorbent resin particles and the fibrous material was 10% by mass), Table 3 shows the results of Production Example B ((the content of the water-absorbent resin particles in the total amount of the water-absorbent resin particles and the fibrous material was 30% by mass), Table 4 shows the results of Production Example C (the content of the water-absorbent resin particles in the total amount of the water-absorbent resin particles and the fibrous material was 60% by mass), and Table 5 shows the results of Production Example D (the content of the water-absorbent resin particles in the total amount of the water-absorbent resin particles and the fibrous material was 100% by mass).

[Table 2]

|  | Ratio of coated resin particles in particles (% by mass) | Diffusion area (cm$^2$) | | | | Ratio of S4 to area of absorber core (%) | Diffusion area change ratio | Leakage (g) | Absorption rate of absorber (seconds) |
|---|---|---|---|---|---|---|---|---|---|
|  |  | S1 | S2 | S3 | S4 |  |  |  |  |
| Example 1 | 20 | 220 | 255 | 262 | 300 | 62 | 0.84 | 0 | 52 |
| Comparative Example 1 | 70 | 197 | 237 | 262 | 310 | 65 | 0.76 | 0 | 63 |

[Table 3]

|  | Ratio of coated resin particles in particles (% by mass) | Diffusion area (cm$^2$) | | | | Ratio of S4 to area of absorber core (%) | Diffusion area change ratio | Leakage (g) | Absorption rate of absorber (seconds) |
|---|---|---|---|---|---|---|---|---|---|
|  |  | S1 | S2 | S3 | S4 |  |  |  |  |
| Comparative Example 2 | 0 | 242 | 287 | 320 | 367 | 77 | 0.77 | 0 | 121 |
| Example 2 | 10 | 275 | 312 | 342 | 380 | 79 | 0.81 | 0 | 105 |
| Example 3 | 20 | 247 | 247 | 265 | 300 | 62 | 0.88 | 0 | 95 |
| Example 4 | 40 | 257 | 270 | 295 | 345 | 72 | 0.82 | 0 | 91 |
| Example 5 | 60 | 245 | 275 | 300 | 332 | 69 | 0.82 | 0 | 106 |
| Example 6 | 20 | 225 | 245 | 287 | 295 | 61 | 0.81 | 0 | 103 |

[Table 4]

|  | Ratio of coated resin particles in particles (% by mass) | Diffusion area (cm$^2$) | | | | Ratio of S4 to area of absorber core (%) | Diffusion area change ratio | Leakage (g) | Absorption rate of absorber (seconds) |
|---|---|---|---|---|---|---|---|---|---|
|  |  | S1 | S2 | S3 | S4 |  |  |  |  |
| Comparative Example 3 | 0 | 247 | 252 | 297 | 357 | 74 | 0.76 | 0 | 212 |
| Example 7 | 20 | 262 | 263 | 290 | 330 | 69 | 0.85 | 0 | 196 |
| Example 8 | 20 | 275 | 275 | 275 | 360 | 75 | 0.87 | 0 | 177 |
| Comparative Example 4 | 20 | 225 | 250 | 280 | 355 | 74 | 0.75 | 0 | 225 |
| Comparative Example 5 | 70 | 337 | 340 | 392 | 467 | 97 | 0.79 | 3 | 224 |

(continued)

| | Ratio of coated resin particles in particles (% by mass) | Diffusion area (cm$^2$) | | | | Ratio of S4 to area of absorber core (%) | Diffusion area change ratio | Leakage (g) | Absorption rate of absorber (seconds) |
|---|---|---|---|---|---|---|---|---|---|
| | | S1 | S2 | S3 | S4 | | | | |
| Comparative Example 10 | 0 | 237 | 214 | 254 | 317 | 66 | 0.79 | 0 | 233 |
| Example 10 | 40 | 257 | 270 | 277 | 362 | 75 | 0.82 | 0 | 205 |
| Example 11 | 20 | 295 | 295 | 310 | 355 | 74 | 089 | 0 | 170 |
| Example 12 | 20 | 280 | 300 | 307 | 357 | 74 | 0.87 | 0 | 165 |
| Example 13 | 20 | 255 | 262 | 262 | 320 | 67 | 0.89 | 0 | 210 |

[Table 5]

| | Ratio of coated resin particles in particles (% by mass) | Diffusion area (cm$^2$) | | | | Ratio of S4 to area of absorber core (%) | Diffusion area change ratio | Leakage (g) | Absorption rate of absorber (seconds) |
|---|---|---|---|---|---|---|---|---|---|
| | | S1 | S2 | S3 | S4 | | | | |
| Comparative Example 6 | 0 | 242 | 242 | 305 | 385 | 80 | 0.70 | 0 | 140 |
| Example 9 | 20 | 275 | 297 | 307 | 387 | 81 | 0.82 | 0 | 115 |
| Comparative Example 7 | 20 | 225 | 250 | 280 | 355 | 74 | 0.75 | 0 | 131 |
| Comparative Example 8 | 70 | 312 | 355 | 380 | 480 | 100 | 0.78 | 2 | 232 |

In each of Production Examples A to D in which the content of the water-absorbent resin particles in the total amount of the water-absorbent resin particles and the fibrous material was the same, the absorption rate was better and leakage was prevented in the absorbers of the examples, as compared to the absorbers of the comparative examples.

**Reference Signs List**

10: absorber core

10a: water-absorbent resin particle

10b: fiber layer

20a, 20b: core wrap sheet

30: liquid permeable sheet

40: liquid impermeable sheet

50: test solution

100: absorbent article

**Claims**

1. An absorber comprising

   an absorber core containing water-absorbent resin particles,
   wherein a diffusion area change ratio measured in the order of the following (1) to (5) is 0.80 to 1.0,

   (1) an absorbent article is produced by disposing, on an upper surface of the absorber, an air-through type polyethylene porous liquid permeable sheet having a basis weight of 22 $g/m^2$ and having the same area as that of the absorber,
   (2) the absorbent article is placed horizontally, a cylinder having an opening with an inner diameter of 3 cm is placed on a center of the absorbent article, 80 ml of artificial urine is injected into the cylinder at one time, and the cylinder is removed,
   (3) an area $S_1$ of a surface of the absorber on which the artificial urine is diffused is measured 20 minutes after the artificial urine injection,
   (4) a second artificial urine injection is performed in the same manner as in (2) 30 minutes after the first artificial urine injection, and a total area $S_2$ of the surface of the absorber on which the artificial urine is diffused is measured 20 minutes after the second artificial urine injection, and
   (5) the operation of (4) is repeated twice more to measure a diffusion area $S_3$ at the time of a third injection and a diffusion area $S_4$ at the time of a fourth injection,

$$\text{diffusion area change ratio} = (S_1 + S_2)/(S_3 + S_4).$$

2. The absorber according to claim 1,
   wherein an effective diffusion area fraction is 95% or less,

$$\text{effective diffusion area fraction (\%)} = (S_4/\text{area of absorber core}) \times 100.$$

3. The absorber according to claim 1 or 2,
   wherein a content of the water-absorbent resin particles is 5% to 100% by mass with respect to a total amount of the water-absorbent resin particles and a fibrous material in the absorber.

4. The absorber according to any one of claims 1 to 3,

   wherein the water-absorbent resin particles comprise water-absorbent resin particles A in which a 2-minute value of a water absorption rate of physiological saline is 2.5 cm or less, and
   a content of the water-absorbent resin particles A is 9% to 65% by mass with respect to a total amount of the water-absorbent resin particles in the absorber.

5. The absorber according to claim 4,
   wherein the water-absorbent resin particles comprise water-absorbent resin particles B in which a 2-minute value of a water absorption rate of physiological saline is 4.0 cm or more.

6. The absorber according to claim 4 or 5,
   wherein a water retention capacity of physiological saline of the water-absorbent resin particles A is 16 to 55 g/g.

7. The absorber according to any one of claims 4 to 6,
   wherein the water-absorbent resin particles A are coated resin particles having a water-insoluble coating layer that coats at least a part of a surface of crosslinked polymer particles.

EP 4 306 092 A1

*Fig.1*

# Fig.2

| | INTERNATIONAL SEARCH REPORT | International application No. |
|---|---|---|
| | | **PCT/JP2022/012368** |

**A. CLASSIFICATION OF SUBJECT MATTER**

*A61F 13/53*(2006.01)i
FI: A61F13/53 300

According to International Patent Classification (IPC) or to both national classification and IPC

**B. FIELDS SEARCHED**

Minimum documentation searched (classification system followed by classification symbols)

A61F13/53

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2-242858 A (SANYO CHEM. IND. LTD.) 27 September 1990 (1990-09-27) page 1, right column, line 17 to page 6, left column, line 11, fig. 1 | 1-4, 6-7 |
| Y | | 5-7 |
| Y | JP 2001-309941 A (OJI PAPER CO., LTD.) 06 November 2001 (2001-11-06) paragraph [0013] | 5-7 |

☐ Further documents are listed in the continuation of Box C.   ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **24 May 2022** | **07 June 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

INTERNATIONAL SEARCH REPORT
Information on patent family members

International application No.

**PCT/JP2022/012368**

| Patent document cited in search report | | | Publication date (day/month/year) | Patent family member(s) | Publication date (day/month/year) |
|---|---|---|---|---|---|
| JP | 2-242858 | A | 27 September 1990 | (Family: none) | |
| JP | 2001-309941 | A | 06 November 2001 | (Family: none) | |

Form PCT/ISA/210 (patent family annex) (January 2015)

**EP 4 306 092 A1**

**Patent documents cited in the description**

- JP H06345819 A
- WO 2011086843 A